# EUROPÄISCHE PATENTSCHRIFT

(11) **EP 0 449 111 B1**
(45) Veröffentlichungstag und Bekanntmachung des Hinweises auf die Patenterteilung: **15.02.1995**
(21) Anmeldenummer: 91104411.3
(22) Anmeldetag: 21.03.1991
(51) Int. Cl.: C07C 211/36, C07C 209/00, C07C 209/26, C07C 209/48

(54) **Verfahren zur Herstellung von 2-(3-Aminopropyl)-cycloalkyl-aminen**
Process for preparing 2-(3-aminopropyl)-cycloalkylamines
Procédé pour la préparation des (amino-3 propyl)-2 cycloalkylamines

(30) Priorität: 30.03.1990 DE 4010254
(43) Veröffentlichungstag der Anmeldung: 02.10.1991
(73) Patentinhaber: BASF Aktiengesellschaft, 67063 Ludwigshafen (DE)
(72) Erfinder: Merger, Franz, Dr., W-6710 Frankenthal (DE); Priester, Claus-Ulrich, Dr., W-6700 Ludwigshafen (DE); Kloppenhoefer, Gerhard, Dr., W-6725 Roemerberg (DE); Harder, Wolfgang, Dr., W-6940 Weinheim (DE); Witzel, Tom, Dr., W-6700 Ludwigshafen (DE); Lermer, Helmut, Dr., W-6700 Ludwigshafen (DE); Koehler, Ulrich, Dr., W-6900 Heidelberg (DE)

(56) Entgegenhaltungen:
- EP-A- 0 042 119
- DE-A- 3 011 656
- US-A- 3 143 570
- CHEMICAL ABSTRACTS, Band 73, Nr. 17, 26. Oktober 1970, Columbus, Ohio, US; IKEYA et al.: "2-(3-Aminopropyl)cyclohexylamine", Seite 324, Zusammenfassung Nr. 87521z & JP-A-4519896

## Beschreibung

Die vorliegende Erfindung betrifft ein neues Verfahren zur Herstellung von 2-(3-Aminopropyl)-cycloalkylaminen aus 2-(2-Cyanoethyl)-cycloalkanonen.

In der JP 70/19896 ist ein Verfahren zur Herstellung von 2-(3-Aminopropyl)-cyclohexylamin beschrieben, nach dem man 2-(2-Cyanoethyl)cyclohexanon mit Ammoniak und Wasserstoff in Gegenwart eines Hydrierkatalysators umsetzt. Nach diesem Verfahren wird durch diskontinuierliche Hydrierung einer Lösung von 2-(2-Cyanoethyl)-cyclohexanon in Methanol in Gegenwart von 10 Gew.-% Raney-Nickel und 14 mol Ammoniak bei 130°C und 110 bar nach einer Reaktionszeit von 5 h 2-(3-Aminopropyl)-cyclohexylamin erhalten. Als Katalysatoren werden Raney-Nickel, Raney-Cobalt, Katalysatoren auf Nickel-, Cobalt- und Kupfer-Basis sowie Edelmetallkatalysatoren (Platin, Palladium, Rhodium und Ruthenium) vorgeschlagen, für die Reaktionstemperatur der Bereich von 100 bis 150°C. Dieses Verfahren hat den Nachteil, daß die Reaktionszeit sehr lang und somit die Raum-Zeit-Ausbeute für eine technische Ausgestaltung des Verfahrens unbefriedigend ist. Weiter war auch die Ausbeute des Verfahrens noch verbesserungsbedürftig (Vergleichsbeispiel 1).

In der EP-A-42 119 schließlich ist ein Verfahren zur Herstellung primärer Mono- und Diamine aus Oxoverbindungen, die gegebenenfalls auch weitere zur Reduktion befähigte Gruppen enthalten können, mit Ammoniak und Wasserstoff in Gegenwart bekannter Hydrierkatalysatoren beschrieben, bei dem vor der Reaktion mit Ammoniak und Wasserstoff in Gegenwart von Hydrierkatalysatoren die Oxo-Verbindungen bei Temperaturen von 10 bis 200°C und Drücken von 1 bis 300 bar einer Vorreaktion mit Ammoniak in Gegenwart von anorganischen und organischen Ionenaustauschern in der Ammoniumform als Iminbildungskatalysatoren unterwirft. Die Anwendung des Verfahrens ist ausschließlich in den Beispielen für die aminierende Hydrierung von 3-Cyano-3,5,5-trimethyl-cyclohexanon (Isophoronnitril) und 2,2,6,6-Tetramethyl-4-piperidon (Triacetonamin) beschrieben. Bei der aminierenden Hydrierung von Isophoronnitril werden durch den Einsatz des organischen Ionenaustauschers Lewatit SP® 120 in der Iminierung geringfügige Ausbeuteverbesserungen gegenüber der nicht-katalysierten Fahrweise erzielt (vgl. Vergleichsbeispiel 3 in EP-A-42 119: 90,3 %. Ausbeute, mit Lewatit SP® 120: 93,9 bis 94,7 %.

Der vorliegenden Erfindung lag daher die Aufgabe zugrunde, ein Verfahren zur Herstellung von 2-(3-Aminopropyl)-cycloalkylaminen aus 2-(2-Cyanoethyl)-cycloalkanonen unter technisch praktikablen Bedingungen bereitzustellen, das technisch befriedigende Ausbeuten und Raum-Zeit-Ausbeuten liefert.

Demgemäß wurde ein neues und verbessertes Verfahren zur Herstellung von 2-(3-Aminopropyl)-cycloalkylaminen der allgemeinen Formel I 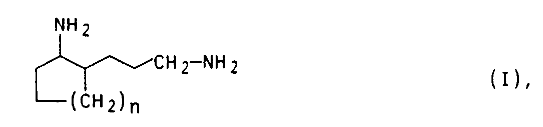
in der der Index n eine ganze Zahl von 1 bis 4 bedeutet, aus 2-(2-Cyanoethyl)-cycloalkanonen der allgemeinen Formel II 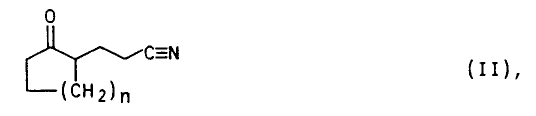
in der der Index n die oben genannte Bedeutung hat, gefunden, welches dadurch gekennzeichnet ist, daß man in zwei räumlich voneinander getrennten Reaktionsräumen
a) die 2-(2-Cyanoethyl)-cycloalkanone der Formel II in einem ersten Reaktionsraum mit überschüssigem Ammoniak an Metallverbindungen mit Lewissäure- oder Brönstedtsäure-Charakter bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an cobalt-, nickel-, ruthenium- und/oder anderen edelmetallhaltigen Katalysatoren gegebenenfalls mit basischen Komponenten oder auf neutralen oder basichen Trägern bei Temperaturen von 60 bis 150°C und Drücken von 50 bis 300 bar hydriert, sowie die neue Verbindung 2-(3-Aminopropyl)-cyclopentylamin.

Das erfindungsgemäße Verfahren läßt sich wie folgt in zwei räumlich voneinander getrennten Reaktionsräumen durchführen:
a) In einer ersten Verfahrensstufe setzt man 2-(2-Cyanoethyl)-cycloalkanone mit überschüssigem Ammoniak bei Temperaturen von 20 bis 150°C, vorzugsweise von 30 bis 130°C, besonders bevorzugt von 50 bis 100°C und Drücken von 15 bis 500 bar, vorzugsweise von 100 bis 350 bar zu den 2-(2-Cyanoethyl)-cycloalkyliminen um. Als acide Heterogenkatalysatoren eignen sich Metallverbindungen mit Lewissäure- oder Brönstedtsäure-Charakter wie z.B. Aluminiumoxid, Siliciumdioxid, Titandioxid, Zirkondioxid, ferner Phosphate, wie z.B. Aluminiumphosphate oder Silikate, wie z.B. amorphe oder kristalline Aluminosilikate. Bevorzugt verwendet man Aluminiumoxid, Titandioxid, Zirkondioxid und Siliciumdioxid, insbesondere Aluminiumoxid und Titandioxid. Die Acidität der Katalysatoren kann gegebenenfalls durch Dotierung mit Halogeniden erhöht werden. So finden z.B. auch halogendotierte Katalysatoren, wie Chlorid auf Aluminiumoxid oder Chlorid auf Titandioxid, Verwendung.
   Bei der Iminierung hält man eine Katalysatorbelastung von 0,01 bis 10, vorzugsweise von 0,05 bis 7, besonders bevorzugt von 0,1 bis 5 kg 2-(2-Cyanoethyl)-cycloalkanon pro kg Katalysator und Stunde ein. Pro Mol 2-(2-Cyanoethyl)-cycloalkanon setzt man bei der Iminierung zweckmäßig, aber nicht zwingend 5 bis 500 Mol NH₃, bevorzugt 10 bis 400 Mol, besonders bevorzugt 20 bis 300 Mol ein. Die Iminierung der 2-(2-Cyanoethyl)-cycloalkanone kann auch in Anwesenheit eines Lösungsmittels, wie z.B. Alkanolen oder Tetrahydrofuran durchgeführt werden.
   Die Iminierung wird bevorzugt kontinuierlich durchgeführt, z.B. in Druckbehältern oder Druckbehälterkaskaden. Nach einer besonders bevorzugten Ausführungsform werden die 2-Cyanoethyl-cycloalkanone und NH₃ durch einen Rohrreaktor geleitet, in dem der Iminierungskatalysator in Form eines festen Bettes angeordnet ist.
   Die Gesamtverweilzeit in Stufe 1 ergibt sich aus der Katalysatorbelastung und der Menge an eingesetztem Ammoniak. Sie liegt zweckmäßig im Bereich von 0,5 bis 120 Minuten, bevorzugt von 1 bis 40, besonders bevorzugt von 1,5 bis 20 Minuten.
b)
   Das so erhaltene Produkt wird in einer zweiten Verfahrensstufe mit 3 bis 10.000 Moläquivalenten Wasserstoff, bevorzugt 4,5 bis 30, gegebenenfalls nach Zufuhr von weiterem Ammoniak, einer katalytischen Hydrierung zugeführt.
   Bei der aminierenden Hydrierung hält man eine Temperatur von 60 bis 150°C, bevorzugt von 70 bis 140°C, besonders bevorzugt von 80 bis 130°C und einen Druck von 50 bis 500 bar, bevorzugt von 100 bis 350 bar, besonders bevorzugt von 150 bis 300 bar ein.
   Die Katalysatorbelastungen liegen zweckmäßig im Bereich von 0,01 bis 5 kg/[kg·h], bevorzugt bei 0,02 bis 2,5, besonders bevorzugt bei 0,05 bis 2 kg/[kg·h].
   Die Hydrierung wird vorzugsweise in flüssigem Ammoniak durchgeführt. Pro Mol 2-(2-Cyanoethyl)-cycloalkylimin setzt man 5 bis 500 Mol NH₃ ein, bevorzugt 10 bis 400, besonders bevorzugt 20 bis 300 Mol. Zweckmäßigerweise wählt man das NH₃-Angebot, das bei der vorgelagerten Herstellung der 2-(2-Cyanoethyl)-cycloalkylimine aus den entsprechenden 2-(2-Cyanoethyl)-cycloalkanonen eingestellt wurde. Der NH₃-Anteil kann jedoch auch vor der Hydrierung durch Zugabe von zusätzlichem NH₃ auf den gewünschten Wert erhöht werden.
   Die aminierende Hydrierung der 2-(2-Cyanoethyl)-cycloalkylimine führt man bevorzugt kontinuierlich z.B. in druckfesten Rührbehältern oder in einer Rührbehälterkaskade durch. In einer besonders bevorzugten Ausführungsform werden Rohrreaktoren eingesetzt, in denen das Produktgemisch aus der Iminierung der 2-(2-Cyanoethyl)-cycloalkanone in Sumpf- oder Rieselfahrweise über ein fest angeordnetes Katalysatorbett geleitet wird.
   Die Verfahrensstufen a und b können ebenfalls in einem Reaktor durchgeführt werden, in dem Iminierungskatalysatoren und Hydrierkatalysatoren in zwei getrennten Schichten angeordnet sind. In diesem Fall führt man die Iminierung zweckmäßigerweise in Gegenwart von Wasserstoff durch.
   Bei kontinuierlicher Fahrweise im Rohrreaktor ohne Rückführung ergibt sich die Gesamtverweilzeit aus der Katalysatorbelastung und der Menge an eingesetztem Ammoniak. Sie liegt im Bereich von 0,5 bis 120 Minuten, bevorzugt von 1 bis 40, besonders bevorzugt bei 1,5 bis 20 Minuten.
   Nach der Hydrierung wird überschüssiges Ammoniak gegebenenfalls unter Druck abgetrennt. Die so erhaltenen 2-(3-Aminopropyl)-cycloalkylamine lassen sich durch fraktionierende Destillation isolieren. Piperidine (z.B. Decahydrochinolin aus 2-(2-Cyanoethyl)-cyclohexanon oder 2-Azabicyclo[4.3.0]nonan aus 2-(2-Cyanoethyl)-cyclopentanon), entstehen nur in untergeordnetem Maße als Nebenprodukte.
   Bei der Hydrierung können prinzipiell alle gängigen Hydrierkatalysatoren eingesetzt werden, die Nickel, Cobalt, Eisen, Kupfer, Ruthenium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/Aluminiumoxide in Frage, bevorzugt werden Hydrierkatalysatoren mit basischen Komponenten, wie Oxide und Hydroxide von Alkali- und Erdalkalimetallen. Besonders bevorzugt sind daher basische Träger, wie z.B. β-Aluminiumoxid oder Mangesiumoxid/Aluminiumoxide. Besonders bevorzugt ist Magnesiumoxid/Aluminiumoxid mit einem Magnesiumoxidanteil von 5 bis 40 %. Dabei kann der Magnesiumoxid und Aluminiumoxide enthaltende Träger amorph sein oder als Spinell vorliegen.
   Besonders bevorzugt verwendet man in der Hydrierung Cobalt oder Ruthenium mit basischer komponente. Diese Katalysatoren erhält man technisch in an sich bekannter Weise. So gewinnt man z.B. Ruthenium auf basischem Träger durch Auftragen von wäßrigen Rutheniumsalz-Lösungen wie Rutheniumchlorid und Rutheniumnitrat auf den entsprechenden Träger. Die Ruthenium-Konzentration auf dem Träger beträgt 0,1 bis 10 %, bevorzugt 0,5 bis 5 %, besonders bevorzugt 1 bis 4 %. Nach Trocknung und gegebenenfalls nach Calcinierung bei Temperaturen von 120 bis 500°C, bevorzugt bei 200 bis 400°C, erfolgt die Aktivierung der Ruthenium-Katalysatoren im Wasserstoffstrom bei Temperaturen von 180 bis 250°C, bevorzugt bei 190 bis 230°C und Drücken von 1 bis 500 bar, bevorzugt von 20 bis 300 bar innerhalb von 1 bis 20 Stunden, bevorzugt 2 bis 10 Stunden.
   Die Ruthenium-Katalysatoren können gegebenenfalls noch weitere Metalle enthalten, wie Palladium oder Eisen. Der Eisengehalt liegt dabei im allgemeinen im Bereich von 0,5 bis 5 %, der Palladiumgehalt im Bereich von 0,1 bis 5 %.
   Die Ruthenium-Katalysatoren zeichnen sich dadurch aus, daß mit diesen besonders hohe Katalysatorbelastungen realisiert und somit besonders hohe Raum-Zeit-Ausbeuten erzielt werden können.
   Die basischen Cobalt-Katalysatoren enthalten mindestens eine basische Komponente wie Li₂O, Na₂O, K₂O, MgO, CaO, SrO oder BaO. Bevorzugt enthalten diese Katalysatoren daneben mindestens eines der Elemente Eisen, Nickel, Mangan, Chrom, Molybdän, Wolfram oder Phosphor. Von besonderem Interesse sind solche Kontakte, die neben Cobalt und einer basischen Komponente mindestens eines der Elemente Eisen, Nickel oder Mangan enthalten. Dabei können die Metalle in metallischer Form oder in Form ihrer Oxide eingesetzt werden. Phosphor liegt praktisch in Form von Phosphorsäure vor.
   Die basische Komponente kann ggf. auch während des Hydrierprozesses zugeführt werden, z.B. als Lösung von Alkali- oder Erdalkalihydroxiden in Wasser.

### Beispiele

### Beispiel 1

Ein vertikaler Rohrreaktor (Durchmesser: 16 mm, Füllhöhe: 22 cm, ölbeheizter Doppelmantel) wurde mit 29,9 g (43 ml) eines Katalysators mit 1,8 % Ruthenium auf Aluminiumoxid (Pural SB) in Form von 1,5-mm-Strängen gefüllt (Katalysatorherstellung durch Porentränkung von Pural SB mit wäßriger Rutheniumchlorid-Lösung und Trocknen bei 120°C). Der Katalysator wurde zur Reduktion bei einem Druck von 60 bar nach schrittweisem Anheben der Temperatur von 150 auf 220°C innerhalb von 2 h unter Durchleiten von 40 Nl/h Wasserstoff 7 h bei 220°C gehalten.

Durch einen vor den Hydrierreaktor geschalteten Rohrreaktor mit einem Volumen von 20 ml, der mit 12,3 g eines mit 1,1 % Chlorid dotierten Aluminiumoxids (0,2 bis 1-mm-Split) gefüllt war, wurden bei einem Druck von 200 bar und einer Temperatur von 100°C stündlich 9,3 ml 2-(2-Cyanoethyl)-cyclohexanon (Reinheit: 98,2 %, 9,1 g, 0,060 mol) und 430 ml (258 g, 15,2 mol) flüssiges Ammoniak gepumpt und anschließend bei einem Druck von 200 bar und einer Temperatur von 90°C zusammen mit 60 Nl/h (2,7 mol) Wasserstoff von unten nach oben durch den Hydrierreaktor geleitet wurden. Der Austrag enthielt laut gaschromatographischer Analyse 61,8 % 2-(3-Aminopropyl)-cyclohexylamin, 33,0 % Decahydrochinolin, 0,1 % Octahydrochinolin und 2,3 % 2-(3-Aminopropyl)-cyclohexanol entsprechend einer Ausbeute an 2-(3-Aminopropyl)-cyclohexylamin von 60,4 % d.Th..

### Beispiel 2

Ein vertikaler Rohrreaktor (Durchmesser: 16 mm, Füllhöhe: 50 cm, ölbeheizter Doppelmantel) wurde mit 173,9 g (95 ml) eines Cobalt-Katalysators mit 5 % Mn₂O₃ in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 46 h schrittweise von 100 auf 320°C erhöht und dann 48 h bei 320°C gehalten.

Durch einen vor den Hydrierreaktor geschalteten Rohrreaktor (Durchmesser: 16 mm, Füllhöhe: 50 cm, ölbeheizter Doppelmantel), der mit 41,6 g (92 ml) eines mit Aerosil 200 verstrangten Y-Zeolithen (HY-Zeolith : Aerosil 200 = 9 : 1, SiO₂/Al₂O₃ = 6 : 1) gefüllt war, werden bei einem Druck von 250 bar und einer Temperatur von 70°C stündlich von unten nach oben 36,7 g 2-Cyanoethyl-cyclohexanon (Reinheit: 96 %, 0,233 mol) und 1475 ml (885 g, 52,1 mol) flüssiges Ammoniak gepumpt. Anschließend werden stündlich 150 Nl/h (6,7 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Nach Entspannen auf Normaldruck wird der Reaktionsaustrag auf den Kopf einer auf 10°C beheizten, 46 cm langen und mit 5 mm Maschendrahtringen gefüllten Kolonne mit einem Durchmesser von 3 cm geleitet und Ammoniak abgetrennt. Nach Destillation des Austrages aus 19,9 h erhält man neben 189,7 g Decahydrochinolin 431,2 g 2-(3-Aminopropyl)-cyclohexylamin entsprechend einer Ausbeute an 2-(3-Aminopropyl)-cyclohexylamin von 59,6 % d.Th..

### Beispiel 3

Ein vertikaler Rohrreaktor (Durchmesser: 16 mm, Füllhöhe: 50 cm, ölbeheizter Doppelmantel) wurde mit 105,3 g (81 ml) eines Katalysators mit 85 % CoO, 10 % CaO und 5 % Mn₂O₃ in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 64,4 g (92 ml) γ-Aluminiumoxid gefüllt war, werden bei einem Druck von 250 bar und einer Temperatur von 70°C stündlich von unten nach oben 55,4 g 2-Cyanoethylcyclohexanon (Reinheit: 96 %, 0,352 mol) und 875 ml (525 g, 30,9 mol) flüssiges Ammoniak gepumpt. Anschließend werden stündlich 200 Nl/h (8,9 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Nach Aufarbeitung wie in Beispiel 2 beschrieben und Destillation des Austrages aus 3,5 h erhält man neben 33,1 g Decahydrochinolin 138,8 g 2-(3-Aminopropyl)-cyclohexylamin entsprechend einer Ausbeute an 2-(3-Aminopropyl)-cyclohexylamin von 71,5 % d.Th..

### Beispiel 4

Beispiel 3 wird wiederholt, wobei durch Reaktor 1 bei einem Druck von 250 bar und einer Temperatur von 70°C stündlich von unten nach oben 29,7 g 2-Cyanoethyl-cyclohexanon (Reinheit: 96 %, 0,189 mol) und 1200 ml (720 g, 42,4 mol) flüssiges Ammoniak gepumpt werden. Anschließend werden stündlich 125 Nl/h (5,6 mol) Wasserstoff zugefahren und der Austrag aus dem vorgeschalteten Iminierungsreaktor bei einem Druck von 250 bar und einer Temperatur von 125°C von unten nach oben durch den Hydrierreaktor gefahren. Nach Aufarbeitung wie in Beispiel 2 beschrieben und Destillation eines Austrages aus 17,8 h erhält man neben 66,4 g Decahydrochinolin 365 g 2-(3-Aminopropyl)-cyclohexylamin. Die Ausbeute an 2-(3-Aminopropyl)-cyclohexylamin beträgt 69,7 % d.Th..

### Beispiel 5

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes CoO mit 5 % Mn₂O₃ und 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 70,0 g (100 ml) γ-Aluminimumoxid in Form von 1,5 mm-Strängen gefüllt war, werden bei einem Druck von 250 bar und einer Temperatur von 80°C stündlich von unten nach oben 10,7 g 2-Cyanoethyl-cyclohexanon (Reinheit: 97,5 %, 0,069 mol) und 500 ml (300 g, 17,6 mol) flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Der Hydrieraustrag enthält laut gaschromatographischer Analyse 86,3 % 2-(3-Aminopropyl)-cyclohexylamin und 7,9 % Decahydrochinolin entsprechend einer Ausbeute an 2-(3-Aminopropyl)-cyclohexylamin von 87,1 % d.Th..

### Beispiel 6

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes (CoO mit 5 % Mn₂O₃ und 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 63,5 g (100 ml) Titandioxid (Anatas) in Form von 1,5 mm-Strängen gefüllt war, werden bei einem Druck von 250 bar und einer Temperatur von 80°C stündlich von unten nach oben 16,0 g 2-Cyanethyl-cyclohexanon (Reinheit: 97,5 %, 0,103 mol) und 51 g (87 ml, 3,0 mol) flüssiges Ammoniak gepumpt. Anschließend werden stündlich 60 Nl/h Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wird der Ammoniak abdestilliert. Der Hydrieraustrag enthält laut gaschromatographischer Analyse 92,5 % 2-(3-Aminopropyl)-cyclohexylamin und 4,5 % Decahydrochinolin. Der Austrag aus 46 Stunden wird gesammelt und durch fraktionierende Destillation über eine 30 cm Füllkörperkolonne (3 mm Glasringe) aufgetrennt. Man erhält 674 g 2-(3-Aminopropyl)-cyclohexylamin entsprechend einer Ausbeute von 91,0 % d.Th..

### Beispiel 7

Ein vertikaler Rohrreaktor (Durchmesser: 16 mm, Füllhöhe: 31 cm, ölbeheizter Doppelmantel) wurde mit 38,3 g (62 ml) eines Katalysators mit 2,6 % Ruthenium auf einem Magnesiumoxid/Aluminiumoxid-Träger (MgO/Al₂O₃ = 10 : 90) in Form von 1 bis 1,5-mm-Split gefüllt (Katalysatorherstellung durch Porentränkung eines MgO/Al₂O₃-Trägers mit wäßriger Rutheniumnitrat-Lösung und Trocknen bei 120°C). Der Katalysator wurde zur Reduktion bei Normaldruck nach schrittweisem Anheben der Temperatur von 100 auf 220°C innerhalb von 6 h unter Durchleiten von 50 Nl/h Wasserstoff 7 h bei 220°C gehalten.

Durch einen vor den Hydrierreaktor geschalteten Rohrreaktor mit einem Volumen von 20 ml, der mit 10,3 g eines Y-Zeolithen (Y-Zeolith mit 0,14 % Na, SiO₂ : Al₂O₃ = 5,8 : 1) in Form von 1 bis 1,25-mm-Split gefüllt war, wurden bei einem Druck von 270 bar und einer Temperatur von 100°C stündlich 11,9 ml 2-(2-Cyanoethyl)-cyclohexanon (Reinheit: 98,2 %, 11,7 g, 0,077 mol) und 430 ml (258 g, 15,2 mol) flüssiges Ammoniak gepumpt und anschließend bei einem Druck von 200 bar und einer Temperatur von 90°C zusammen mit 60 Nl/h (2,7 mol) Wasserstoff von unten nach oben durch den Hydrierreaktor geleitet. Der Austrag enthielt laut gaschromatographischer Analyse 84,1 % 2-(3-Aminopropyl)-cyclohexylamin, 11,4 % Decahydrochinolin und 0,5 % 2-(3-Aminopropyl)-cyclohexanol entsprechend einer Ausbeute an 2-(3-Aminopropyl)-cyclohexylamin von 84,4 % d.Th..

### Beispiel 8

Ein vertikaler Rohrreaktor (Durchmesser: 16 mm, Füllhöhe: 15 cm, ölbeheizter Doppelmantel) wurde mit 24,8 g (30,2 ml) eines Katalysators mit 2,2 % Ruthenium auf einem Magnesiumoxid/Aluminiumoxid-Träger (10/90) in Form von 1 bis 1,5-mm-Split gefüllt (Katalysatorherstellung durch Porentränkung eines Magnesiumoxid/Aluminiumoxid-Trägers mit wäßriger Rutheniumnitrat-Lösung und Trocknen bei 120°C). Der Katalysator wurde zur Reduktion bei Normaldruck nach schrittweisem Anheben der Temperatur von 100 auf 220°C unter Durchleiten von 60 Nl/h Wasserstoff 7 h bei 220°C gehalten.

Durch einen vor den Hydrierreaktor geschalteten Rohrreaktor mit einem Volumen von 60 ml, der mit 38,1 g Titandioxid (Anatas) in Form von 1,5 mm-Strängen gefüllt war, wurden bei einem Druck von 220 bar und einer Temperatur von 100°C stündlich 9,2 ml 2-(2-Cyanoethyl)-cyclohexanon (Reinheit: 98,2 %, 9,0 g, 0,060 mol) und 270 ml (162 g, 9,5 mol) flüssiges Ammoniak gepumpt. Der Austrag wurde anschließend bei einem Druck von 202 bar und einer Temperatur von 90°C unter gleichzeitigem Durchleiten von 30 Nl/h (1,3 mol) Wasserstoff von oben nach unten durch den Hydrierreaktor geleitet. Nach Entspannen auf Normaldruck wurde der Reaktionsaustrag auf den Kopf einer auf 40°C beheizten, 20 cm langen und mit 8 mm Glasringen gefüllten Kolonne geleitet, durch die im Gegenstrom stündlich 40 l Stickstoff geblasen wurden. Am Kolonnensumpf verblieben stündlich 32,7 g gestrippter Reaktionsaustrag, der laut gaschromatographischer Analyse 83,0 % 2-(3-Aminopropyl)-cyclohexylamin, 12,6 % Decahydrochinolin, 1,1 % Octahydrochinolin und 0,6 % 2-(3-Aminopropyl)-cyclohexanol enthielt. Die Ausbeute an 2-(3-Aminopropyl)-cyclohexylamin beträgt 83,1 % d.Th..

### Beispiel 9

Beispiel 8 wurde wiederholt, wobei durch den Hydrierreaktor 60 Nl/h (2,7 mol) statt 30 Nl/h Wasserstoff geleitet wurden. Aufarbeitung wie in Beispiel 7 beschrieben lieferte einen Reaktionsaustrag, der laut gaschromatographischer Analyse 78,4 % 2-(3-Aminopropyl)-cyclohexylamin, 17,8 % Decahydrochinolin, 0,3 % Octahydrochinolin und 1,5 % 2-(3-Aminopropyl)-cyclohexanol enthielt. Die Ausbeute an 2-(3-Aminopropyl)-cyclohexylamin beträgt 78,1 % d.Th..

### Beispiel 10

Beispiel 8 wurde wiederholt, wobei durch den Iminierungsreaktor bei einem Druck von 219 bar und einer Temperatur von 100°C stündlich 8,7 ml 2-(2-Cyanoethyl)-cyclohexanon (Reinheit: 98,2 %, 8,5 g, 0,056 mol) und 600 ml (360 g, 21,2 mol) flüssiges Ammoniak gepumpt und anschließend bei einem Druck von 203 bar und einer Temperatur von 120°C zusammen mit 60 Nl/h (2,7 mol) Wasserstoff von oben nach unten durch den Hydrierreaktor geleitet wurden. Der Austrag enthält laut gaschromatographischer Analyse 83,0 % 2-(3-Aminopropyl)-cyclohexylamin, 14,8 % Decahydrochinolin und 0,1 % 2-(3-Aminopropyl)-cyclohexanol entsprechend einer Ausbeute an 2-(3-Aminopropyl)-cyclohexylamin von 83 % d.Th..

### Beispiel 11

Beispiel 8 wurde wiederholt, wobei durch den Iminierungsreaktor bei einem Druck von 212 bar und einer Temperatur von 100°C stündlich 18,4 ml 2-(2-Cyanoethyl)-cyclohexanon (Reinheit: 98,2 %, 18 g, 0,119 mol) und 400 ml (240 g, 14,1 mol) flüssiges Ammoniak gepumpt und anschließend bei einem Druck von 204 bar und einer Temperatur von 120°C zusammen mit 60 Nl/h (2,7 mol) Wasserstoff von oben nach unten durch den Hydrierreaktor geleitet wurden. Der Austrag enthält laut gaschromatographischer Analyse 75,7 % 2-(3-Aminopropyl)-cyclohexylamin, 21,6 % Decahydrochinolin und 0,6 % 2-(3-Aminopropyl)-cyclohexanol entsprechend einer Ausbeute an 2-(3-Aminopropyl)-cyclohexylamin von 75,1 % d.Th..

### Beispiel 12

Beispiel 8 wurde wiederholt, wobei durch den Iminierungsreaktor bei einem Druck von 220 bar und einer Temperatur von 100°C stündlich 28,8 ml 2-(2-Cyanoethyl)-cyclohexanon (Reinheit: 98,2 %, 28,2 g, 0,187 mol) und 460 ml (276 g, 16,2 mol) flüssiges Ammoniak gepumpt und anschließend bei einem Druck von 203 bar und einer Temperatur von 120°C zusammen mit 60 Nl/h (2,7 mol) Wasserstoff von oben nach unten durch den Hydrierreaktor geleitet wurden. Der Austrag enthält laut gaschromatographischer Analyse 74,1 % 2-(3-Aminopropyl)-cyclohexylamin, 22,3 % Decahydrochinolin und 1,1 % 2-(3-Aminopropyl)-cyclohexanol entsprechend einer Ausbeute an 2-(3-Aminopropyl)-cyclohexylamin von 73,4 % d.Th..

### Beispiel 13

Beispiel 8 wurde wiederholt, wobei durch den Iminierungsreaktor bei einem Druck von 220 bar und einer Temperatur von 100°C stündlich 29,1 ml 2-(2-Cyanoethyl)-cyclohexanon (Reinheit: 98,2 %, 28,5 g, 0,188 mol) und 460 ml (276 g, 16,2 mol) flüssiges Ammoniak gepumpt und anschließend bei einem Druck von 200 bar und einer Temperatur von 120°C zusammen mit 60 Nl/h (2,7 mol) Wasserstoff von oben nach unten durch den Hydrierreaktor geleitet wurden. Nach Entspannen auf Normaldruck wurde der Reaktionsaustrag auf den Kopf einer auf 40°C beheizten, 20 cm langen und mit 8 mm Glasringen gefüllten Kolonne geleitet, durch die im Gegenstrom stündlich 40 l Stickstoff geblasen wurden. Am Kolonnensumpf verblieben stündlich 32,7 g gestrippter Reaktionsaustrag, der laut gaschromatographischer Analyse 70,7 % 2-(3-Aminopropyl)-cyclohexylamin, 25,5 % Decahydrochinolin, 0,2 % Octahydrochinolin und 1 % 2-(3-Aminopropyl)-cyclohexanol enthielt. Der Austrag aus 5,6 h (183 g) wurde durch fraktionierende Destillation aufgetrennt. Man erhielt neben 3,4 g Destillationsrückstand und 34,4 g Decahydrochinolin (Kp₁₀ = 60 bis 73°C) 112,8 g 2-(3-Aminopropyl)-cyclohexylamin (Kp_{0,5} = 87 bis 90°C) entsprechend einer Ausbeute an 2-(3-Aminopropyl)-cyclohexylamin von 68,1 % d.Th..

### Beispiel 14

Beispiel 8 wurde wiederholt, wobei durch den Iminierungsreaktor bei einem Druck von 221 bar und einer Temperatur von 100°C stündlich 53,2 ml 2-(2-Cyanoethyl)-cyclohexanon (Reinheit: 98,2 %, 52,1 g, 0,345 mol) und 600 ml (360 g, 21,2 mol) flüssiges Ammoniak gepumpt und anschließend bei einem Druck von 203 bar und einer Temperatur von 120°C zusammen mit 120 Nl/h (5,4 mol) Wasserstoff von oben nach unten durch den Hydrierreaktor geleitet wurden. Der Austrag enthielt laut gaschromatographischer Analyse 66,2 % 2-(3-Aminopropyl)-cyclohexylamin, 28,2 % Decahydrochinolin, 1,7 % Octahydrochinolin und 1,3 % 2-(3-Aminopropyl)-cyclohexanol entsprechend einer Ausbeute an 2-(3-Aminopropyl)-cyclohexylamin von 65,0 % d.Th..

### Beispiel 15

Ein vertikaler Rohrreaktor (Durchmesser: 16 mm, Füllhöhe: 24 cm, ölbeheizter Doppelmantel) wurde mit 40,4 g (47 ml) eines Katalysators mit 2,7 % Ruthenium auf einem Magnesiumoxid/Aluminiumoxid-Träger (MgO/Al₂O₃ = 10/90) in Form von 1 bis 1,5-mm-Split gefüllt (Katalysatorherstellung durch Porentränkung eines MgO/Al₂O₃-Trägers mit wäßriger Rutheniumchlorid-Lösung und Trocknen bei 120°C). Der Katalysator wurde zur Reduktion bei Normaldruck nach schrittweisem Anheben der Temperatur von 100 auf 220°C innerhalb von 6 h unter Durchleiten von 50 Nl/h Wasserstoff 7 h bei 220°C gehalten.

Durch einen vor den Hydrierreaktor geschalteten Rohrreaktor mit einem Volumen von 20 ml, der mit 11,4 g eines mit Aerosil 200 verstrangten Y-Zeolithen (Y-Zeolith mit 0,22 % Na, Y-Zeolith : Aerosil 200 = 90 : 10, SiO₂ : Al₂O₃ = 6 : 1) in Form von 1 bis 1,25-mm-Split gefüllt war, wurden bei einem Druck von 310 bar und einer Temperatur von 100°C stündlich 11,4 ml 2-(2-Cyanoethyl)-cyclohexanon (Reinheit: 98,2 %, 11,1 g, 0,074 mol) und 430 ml (258 g, 15,2 mol) flüssiges Ammoniak gepumpt und anschließend bei einem Druck von 300 bar und einer Temperatur von 90°C zusammen mit 60 Nl/h (2,7 mol) Wasserstoff von oben nach unten durch den Hydrierreaktor geleitet. Der Austrag enthielt laut gaschromatographischer Analyse 73,6 % 2-(3-Aminopropyl)-cyclohexylamin, 24,2 % Decahydrochinolin und 0,3 % 2-(3-Aminopropyl)-cyclohexanol entsprechend einer Ausbeute an 2-(3-Aminopropyl)-cyclohexylamin von 72,8 % d.Th..

### Beispiel 16

Ein vertikaler Rohrreaktor (Durchmesser: 16 mm, Füllhöhe: 30 cm, ölbeheizter Doppelmantel) wurde mit 38,9 g (60 ml) eines Katalysators mit 5,4 % Ruthenium und 0,8 % Eisen auf einem Magnesiumoxid/Aluminiumoxid-Träger (MgO/Al₂O₃ = 10/90) in Form von 1 bis 1,5-mm-Split gefüllt (Katalysatorherstellung durch Porentränkung eines MgO/Al₂O₃-Trägers mit wäßriger Eisen- und Rutheniumnitrat-Lösung und Trocknen bei 120°C). Der Katalysator wurde zur Reduktion bei Normaldruck nach schrittweisem Anheben der Temperatur von 100 auf 220°C innerhalb von 6 h unter Durchleiten von 50 Nl/h Wasserstoff 7 h bei 220°C gehalten.

Durch einen vor den Hydrierreaktor geschalteten Rohrreaktor mit einem Volumen von 60 ml, der mit 38,1 g Titandioxid (Anatas) in Form von 1,5-mm-Strängen gefüllt war, wurden bei einem Druck von 280 bar und einer Temperatur von 80°C stündlich 24,2 ml 2-(2-Cyanoethyl)-cyclohexanon (Reinheit: 98,2 %, 23,7 g, 0,157 mol) und 430 ml (258 g, 15,2 mol) flüssiges Ammoniak gepumpt. Der Austrag wurde anschließend bei einem Druck von 200 bar und einer Temperatur von 90°C zusammen mit 60 Nl/h (2,7 mol) Wasserstoff von unten nach oben durch den Hydrierreaktor geleitet. Der Austrag enthielt laut gaschromatographischer Analyse 76,4 % 2-(3-Aminopropyl)-cyclohexylamin, 18,2 % Decahydrochinolin, 0,4 % Octahydrochinolin und 0,9 % 2-(3-Aminopropyl)-cyclohexanol entsprechend einer Ausbeute an 2-(3-Aminopropyl)-cyclohexylamin von 76 % d. Th..

### Beispiel 17

Ein vertikaler Rohrreaktor (Durchmesser: 16 mm, Füllhöhe: 15 cm, ölbeheizter Doppelmantel) wurde mit 25,3 g (30 ml) eines Katalysators mit 2,2 % Ruthenium und 3,4 % Palladium auf einem Magnesiumoxid/Aluminiumoxid-Träger (MgO/Al₂O₃ = 10/90) in Form von 1 bis 1,5-mm-Split gefüllt (Katalysatorherstellung durch Porentränkung eines MgO/Al₂O₃-Trägers mit wäßriger Ruthenium- und Palladiumnitrat-Lösung und Trocknen bei 120°C). Der Katalysator wurde zur Reduktion bei Normaldruck nach schrittweisem Anheben der Temperatur von 100 auf 220°C innerhalb von 6 h unter Durchleiten von 60 Nl/h Wasserstoff 7 h bei 220°C gehalten.

Durch einen vor den Hydrierreaktor geschalteten Rohrreaktor (Durchmesser: 16 mm, 15 cm, ölbeheizter Doppelmantel), der mit 20,3 g (29 ml) γ-Aluminiumoxid in Form von 1,5-mm-Strängen gefüllt war, wurden bei einem Druck von 230 bar und einer Temperatur von 100°C stündlich 20,3 ml 2-(2-Cyanoethyl)-cyclohexanon (Reinheit: 98,2 %, 20,3 g, 0,132 mol) und 600 ml (360 g, 21,2 mol) flüssiges Ammoniak gepumpt und anschließend bei einem Druck von 210 bar und einer Temperatur von 120°C zusammen mit 60 Nl/h (2,7 mol) Wasserstoff von oben nach unten durch den Hydrierreaktor geleitet. Der Austrag enthielt laut gaschromatographischer Analyse 69,5 % 2-(3-Aminopropyl)-cyclohexylamin, 25,4 % Decahydrochinolin und 2,2 % 2-(3-Aminopropyl)-cyclohexanol entsprechend einer Ausbeute an 2-(3-Aminopropyl)-cyclohexylamin von 68,6 % d.Th..

### Beispiel 18

Ein vertikaler Rohrreaktor (Durchmesser: 16 mm, Füllhöhe: 50 cm, ölbeheizter Doppelmantel) wurde mit 76,9 g (102 ml) eines Katalysators mit 5 % Ruthenium auf einem Magnesiumoxid/Aluminiumoxid-Träger (30/70) in Form von 1 bis 1,5-mm-Split gefüllt (Katalysatorherstellung durch Porentränkung eines Magnesiumoxid/Aluminiumoxid-Trägers mit wäßriger Rutheniumnitrat-Lösung und Trocknen bei 120°C). Zur Reduktion des Katalysators wurde bei 100 bar unter gleichzeitigem Durchleiten von 150 Nl/h Wasserstoff die Temperatur innerhalb von 7 h schrittweise von 100 auf 220°C erhöht und dann 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 67,2 g (96 ml) γ-Aluminiumoxid in Form von 1,5-mm-Strängen gefüllt war, werden bei einem Druck von 250 bar und einer Temperatur von 70°C stündlich von unten nach oben 42,6 g 2-Cyanoethyl-cyclohexanon (Reinheit: 96 %, 0,271 mol) und 1283 ml (770 g, 45,3 mol) flüssiges Ammoniak gepumpt. Anschließend werden stündlich 90 Nl/h (4,0 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Der Austrag aus 18 h enthält laut quantitativer gaschromatographischer Analyse 541,9 g 2-(3-Aminopropyl)-cyclohexylamin. Die Ausbeute an 2-(3-Aminopropyl)-cyclohexylamin beträgt 71,5 % d.Th..

### Beispiel 19

Ein vertikaler Rohrreaktor (Durchmesser: 16 mm, Füllhöhe: 50 cm, ölbeheizter Doppelmantel) wurde mit 81,3 g (103 ml) eines Katalysators mit 3 % Ruthenium auf einem Calciumoxid/Aluminiumoxid-Träger (10/90) in Form von 1,5-mm-Strängen gefüllt (Katalysatorherstellung durch Diffusionstränkung eines Calciumoxid/Aluminiumoxid-Trägers mit wäßriger Rutheniumnitrat-Lösung und Trocknen bei 100°C). Zur Reduktion des Katalysators wurde bei 100 bar unter gleichzeitigem Durchleiten von 150 Nl/h Wasserstoff die Temperatur innerhalb von 7 h schrittweise von 100 auf 220°C erhöht und dann 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 67,2 g (96 ml) γ-Aluminiumoxid in Form von 1,5-mm-Strängen gefüllt war, werden bei einem Druck von 250 bar und einer Temperatur von 70°C stündlich von unten nach oben 37,1 g 2-Cyanoethyl-cyclohexanon (Reinheit: 96 %, 0,236 mol) und 1290 ml (774 g, 45,5 mol) flüssiges Ammoniak gepumpt. Anschließend werden stündlich 120 Nl/h (5,4 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Der Austrag aus 20,6 h enthält laut quantitativer gaschromatographischer Analyse 536,1 g 2-(3-Aminopropyl)-cyclohexylamin. Die Ausbeute an 2-(3-Aminopropyl)-cyclohexylamin beträgt 70,5 % d.Th..

### Beispiel 20

Ein vertikaler Rohrreaktor (Durchmesser: 16 mm, Füllhöhe: 50 cm, ölbeheizter Doppelmantel) wurde mit 90,1 g (87 ml) eines Katalysators mit 3 % Ruthenium auf β-Aluminiumoxid in Form von 1,2-mm-Strängen gefüllt (Katalysatorherstellung durch Porentränkung von β-Aluminiumoxid mit wäßriger Rutheniumnitrat-Lösung und Trocknen bei 120°C). Zur Reduktion des Katalysators wurde bei 100 bar unter gleichzeitigem Durchleiten von 150 Nl/h Wasserstoff die Temperatur innerhalb von 7 h schrittweise von 100 auf 220°C erhöht und dann 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 67,2 g (96 ml) γ-Aluminiumoxid in Form von 1,5-mm-Strängen gefüllt war, werden bei einem Druck von 250 bar und einer Temperatur von 70°C stündlich von unten nach oben 22,6 g 2-Cyanoethyl-cyclohexanon (Reinheit: 96 %, 0,144 mol) und 1086 ml (652 g, 38,3 mol) flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Nach Aufarbeitung und Destillation eines Austrages aus 16,3 h erhält man neben 43,5 g Decahydrochinolin 291,7 g 2-(3-Aminopropyl)-cyclohexylamin. Die Ausbeute an 2-(3-Aminopropyl)-cyclohexylamin beträgt 79,8 % d.Th..

### Beispiel 21

Ein vertikaler Rohrreaktor (Durchmesser: 16 mm, Füllhöhe: 24 cm, ölbeheizter Doppelmantel) wurde mit 43,3 g (42 ml) eines Katalysators mit 3 % Ruthenium auf β-Aluminiumoxid in Form von 1,2-mm-Strängen gefüllt (Katalysatorherstellung durch Porentränkung von β-Aluminiumoxid mit wäßriger Rutheniumnitrat-Lösung und Trocknen bei 120°C). Zur Reduktion des Katalysators wurde bei 100 bar unter gleichzeitigem Durchleiten von 150 Nl/h Wasserstoff die Temperatur innerhalb von 7 h schrittweise von 100 auf 220°C erhöht und dann 9 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 67,2 g (96 ml) γ-Aluminiumoxid in Form von 1,5-mm-Strängen gefüllt war, werden bei einem Druck von 250 bar und einer Temperatur von 70°C stündlich von unten nach oben 11,0 g 2-Cyanoethyl-cyclopentanon (Reinheit: 77,0 %, 0,062 mol) und 535 ml (321 g, 18,9 mol) flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Der Austrag aus 45,4 h wird durch fraktionierende Destillation aufgetrennt. Man erhält 91,2 g 1-Azabicyclo(4.3.0)nonan (Kp₅₀ = 80°C) und 224,4 g 2-(3-Aminopropyl)-cyclopentylamin (Kp₂₈ = 122°C . Die Ausbeute an 2-(3-Aminopropyl)-cyclopentylamin beträgt 56,3 % d.Th.

### Vergleichsbeispiel 1

In einem 300 ml-Autoklav gibt man 37,8 g 2-Cyanoethylcyclohexanon (Reinheit: 98,2 %), 25,5 ml Methanol und 3,8 g Raney-Cobalt, spült mit Stickstoff und drückt 60,0 g flüssigen Ammoniak zu. Nach Aufpressen von 110 bar Wasserstoff erhitzt man unter Rühren auf 130°C; sobald der Druck unter 110 bar abfällt, wird nachgepreßt. Nach 5 h ist die Wasserstoffaufnahme beendet. Abdestillieren von Ammoniak und Methanol liefert ein Reaktionsgemisch, welches laut GC 22,1 % 2-(3-Aminopropyl)-cyclohexylamin und 72,1 % Decahydrochinolin enthält. Dies entspricht einer Diamin-Ausbeute von 20,4 %.

### Vergleichsbeispiel 2

Ein vertikaler Rohrreaktor (Durchmesser: 9 mm, ölbeheizter Doppelmantel) wurde mit 38,8 g (25 ml) eines handelsüblichen Cobalt-Katalysators mit 5 % Mn₃O₄ in Form von 2 bis 3-mm-Split gefüllt. Der Katalysator wurde zur Reduktion bei einem Druck von 60 bar nach schrittweisem Anheben der Temperatur von 110 auf 180°C innerhalb von 2 h unter Durchleiten von 25 Nl/h Wasserstoff 1 h bei 180°C und 15 h bei 200°C gehalten.

Bei einer Temperatur von 75°C, einem Druck von 98 bar und einer mittleren Verweilzeit von 25,6 Minuten wurden stündlich 16 Nl/h (0,7 mol) Wasserstoff, 7 ml 2-(2-Cyanoethyl)-cyclohexanon (Reinheit: 98,2 %, 6,9 g, 0,046 mol) und 37 ml (22 g, 1,3 mol) flüssiges Ammoniak von unten nach oben durch den Hydrierreaktor gepumpt. Der Austrag enthielt laut gaschromatographischer Analyse 72 % Decahydrochinolin, 4,1 % Octahydrochinolin, 7,4 % 2-(3-Aminopropyl)-cyclohexanol und 13,1 % 2-(3-Aminopropyl)-cyclohexylamin entsprechend einer Ausbeute an 2-(3-Aminopropyl)-cyclohexylamin von 12,6 % d.Th..

### Vergleichsbeispiel 3

Vergleichsbeispiel 2 wurde bei einer Temperatur von 100°C, einem Druck von 98 bar und einer mittleren Verweilzeit von 4,9 Minuten wiederholt, wobei stündlich 15 Nl/h (0,7 mol) Wasserstoff, 6,6 ml 2-(2-Cyanoethyl)-cyclohexanon (Reinheit: 98,2 %, 6,5 g, 0,043 mol) und 216 ml (129 g, 7,6 mol) flüssiges Ammoniak von unten nach oben durch den Hydrierreaktor gepumpt wurden. Der Reaktoraustrag enthielt nach gaschromatographischer Analyse 77,9 % Decahydrochinolin, 2,0 % Octahydrochinolin, 11,3 % 2-(3-Aminopropyl)-cyclohexanol und 6,0 % 2-(3-Aminopropyl)-cyclohexylamin entsprechend einer Ausbeute an 2-(3-Aminopropyl)-cyclohexylamin von 5,6 % d.Th..

### Vergleichsbeispiel 4

Ein vertikaler Rohrreaktor (Durchmesser: 9 mm, ölbeheizter Doppelmantel) wurde mit 24,8 g (25 ml) eines Katalysators mit 14,5 % Cobalt auf Aluminiumoxid in Form von 2 bis 3-mm-Split gefüllt. Zur Aktivierung des Katalysators wurde bei Normaldruck unter Durchleiten von 13 Nl/h Wasserstoff die Temperatur innerhalb von 24 h schrittweise von 100 auf 300°C angehoben und anschließend unter Durchleiten von 25 Nl/h Wasserstoff 5 h bei 300°C gehalten.

Bei einer Temperatur von 110°C, einem Druck von 98 bar und einer mittleren Verweilzeit von 7,1 Minuten wurden stündlich 12 Nl/h (0,5 mol) Wasserstoff, 5,2 ml 2-(2-Cyanoethyl)-cyclohexanon (Reinheit: 98,2 %, 5,1 g, 0,034 mol) und 172 ml (103 g, 6,1 mol) flüssiges Ammoniak von unten nach oben durch den Hydrierreaktor gepumpt. Der Reaktoraustrag enthielt laut gaschromatographischer Analyse 40,2 % Decahydrochinolin, 21,2 % Octahydrochinolin, 0,7 % 2-(3-Aminopropyl)-cyclohexanol und 34,2 % 2-(3-Aminopropyl)-cyclohexylamin entsprechend einer Ausbeute an 2-(3-Aminopropyl)-cyclohexylamin von 32,2 % d.Th..

### Vergleichsbeispiel 5

Ein vertikaler Rohrreaktor (Durchmesser: 14 mm, Füllhöhe: 43 cm, ölbeheizter Doppelmantel) wurde mit 35,7 g (59,5 ml) eines Katalysators mit 1,8 % Ruthenium auf Aluminiumoxid (Katalysatorherstellung durch Diffusionstränkung von Aluminiumoxid mit wäßriger Rutheniumchlorid-Lösung und Trocknen bei 70°C) gefüllt. Zur Reduktion wurde bei einem Druck von 60 bar 50 Nl/h Wasserstoff durch den Reaktor geleitet, die Temperatur schrittweise innerhalb von 7 h von 100 auf 220°C angehoben und 6 h bei 220°C gehalten.

Bei einer Temperatur von 90°C, einem Druck von 90 bar und einer mittleren Verweilzeit von 4,0 Minuten wurden stündlich 60 Nl/h (2,7 mol) Wasserstoff, 16,1 ml 2-(2-Cyanoethyl)-cyclohexanon (Reinheit: 98,2 %, 15,8 g, 0,104 mol) und 645 ml (387 g, 22,8 mol) flüssiges Ammoniak von unten nach oben durch den Hydrierreaktor gepumpt. Der Reaktoraustrag enthielt nach gaschromatographischer Analyse 44,9 % Decahydrochinolin, 11,6 % Octahydrochinolin, 6,5 % 2-(3-Aminopropyl)-cyclohexanol und 32,7 % 2-(3-Aminopropyl)-cyclohexylamin entsprechend einer Ausbeute an 2-(3-Aminopropyl)-cyclohexylamin von 31,0 % d.Th..

### Vergleichsbeispiel 6

Vergleichsbeispiel 5 wurde bei einem Druck von 200 bar wiederholt. Der Austrag enthielt jetzt 69,1 % Decahydrochinolin, 18,3 % 2-(3-Aminopropyl)-cyclohexanol und 10,5 % 2-(3-Aminopropyl)-cyclohexylamin entsprechend einer Diamin-Ausbeute von 9,9 % d.Th..

## Patentansprüche

1. Verfahren zur Herstellung von 2-(3-Aminopropyl)-cycloalkylaminen der allgemeinen Formel I 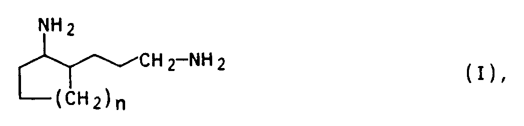 in der der Index n eine ganze Zahl von 1 bis 4 bedeutet, aus 2-(2-Cyanoethyl)-cycloalkanonen der allgemeinen Formel II 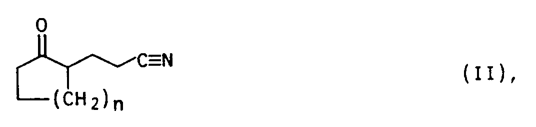 in der der Index n die oben genannte Bedeutung hat, dadurch gekennzeichnet, daß man in zwei räumlich voneinander getrennten Reaktionsräumen
a) die 2-(2-Cyanoethyl)-cycloalkanone der Formel II in einem ersten Reaktionsraum mit überschüssigem Ammoniak an Metallverbindungen mit Lewissäure- oder Brönstedtsäure-Charakter bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an cobalt-, nickel-, ruthenium- und/oder anderen edelmetallhaltigen Katalysatoren, gegebenenfalls mit basischen Komponenten oder auf neutralen oder basischen Trägern bei Temperaturen von 60 bis 150°C und Drücken von 50 bis 500 bar hydriert.

## Claims

1. A process for the preparation of 2-(3-aminopropyl)-cycloalkylamines of the general formula I 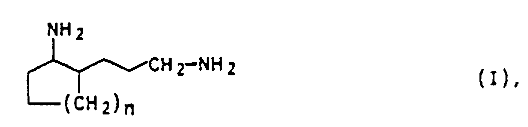 where n is an integer from 1 to 4, from a 2-(2-cyanoethyl)-cycloalkanone of the general formula II 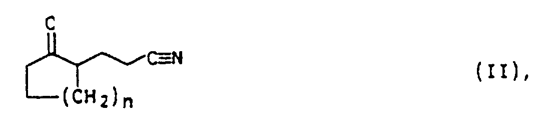 where n has the abovementioned meanings, wherein, in two reaction zones spatially separated from one another,
a) the 2-(2-cyanoethyl)-cycloalkanone of the formula II is reacted, in a first reaction zone, with excess ammonia over a metal compound having the character of a Lewis acid or Brönsted acid at from 20 to 150°C and from 15 to 500 bar and
b) the resulting reaction product is hydrogenated, in a second reaction zone, with hydrogen in the presence of excess ammonia over a catalyst containing cobalt, nickel, ruthenium and/or other noble metals, with or without basic components or on a neutral or basic carrier, at from 60 to 150°C and from 50 to 500 bar.

## Revendications

1. Procédé de préparation de 2-(3-aminopropyl)-cycloalkylamines de la formule générale I 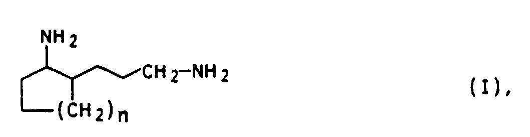 dans laquelle l'indice n représente un nombre entier dont la valeur varie de 1 à 4, au départ de 2-(2-cyanoéthyl)-cycloalcanones de la formule générale II 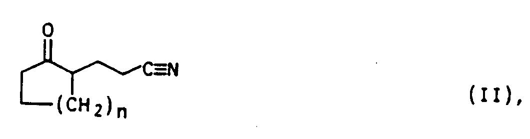 dans laquelle l'indice n possède les significations qui lui ont été attribuées ci-dessus, caractérisé en ce que l'on fait réagir, dans deux enceintes de réaction mutuellement séparées dans l'espace,
a) les 2-(2-cyanoéthyl)-cycloalcanones de la formule II dans une première enceinte de réaction avec de l'ammoniac excédentaire sur des composés de métaux à caractère d'acide de Lewis ou à caractère d'acide de Brönstedt, à des températures de 20 à 150°C et sous des pressions de 15 à 500 bars et
b) on hydrogène les produits de réaction ainsi formés, dans une seconde enceinte de réaction, à l'aide d'hydrogène et en présence d'ammoniac excédentaire sur des catalyseurs contenant du cobalt, du nickel, du ruthénium et/ou d'autres métaux nobles, éventuellement avec des composants basiques, ou sur des supports, neutres ou basiques, à des températures de 60 à 150°C et sous des pressions de 50 à 500 bars.
